# EUROPEAN PATENT APPLICATION

(11) **EP 4 517 488 A1**
(43) Date of publication of application: **05.03.2025**
(21) Application number: 23193673.3
(22) Date of filing: 28.08.2023
(51) Int. Cl.: G06F 3/01, A61B 6/46, G16H 40/63, A61B 5/055, A61B 6/03

(54) **SYSTEM AND METHOD FOR GESTURE CONTROL OF A MEDICAL IMAGING DEVICE**

(71) Applicant: Koninklijke Philips N.V., 5656 AG Eindhoven (NL)
(72) Inventor: FORTHMANN, Peter, Eindhoven (NL); VERNICKEL, Peter, Eindhoven (NL); LIPS, Oliver, 5656AG Eindhoven (NL); PREVRHAL, Sven Peter, Eindhoven (NL)
(74) Representative: Philips Intellectual Property & Standards

(57) **Abstract**

Some embodiments are directed to a system and method for controlling a medical imaging device via user-performed gestures. The medical imaging device comprises a vibration sensor positioned to detect a gesture performed by a user on a surface of the medical imaging device by detecting a vibration propagated through the surface from the gesture to the vibration sensor. A processor system is provided and configured to receive a sensor signal from the vibration sensor and to derive a command signal from the sensor signal to control the medical imaging device.

## Description

### FIELD OF THE INVENTION

The presently disclosed subject matter relates to a system for gesture control of a medical imaging device, to a method for controlling a medical imaging device using gestures, and to a computer-readable medium.

### BACKGROUND OF THE INVENTION

Medical imaging devices are omnipresent in daily clinical practice. For example, various medical treatments rely on the use of scanning devices such as Magnetic Resonance Imaging (MRI), Computed Tomography (CT), Single Photon Emission Computed Tomography (SPECT), Computed Radiography (CR) systems, X-ray apparatuses, and Positron Emission Tomography (PET) scanners, to identify and characterize medical abnormalities, plan interventions, place stents, etc.

It is known to control medical imaging devices using a control panel, for example a physical control panel or a control panel displayed on a graphical user interface. Using such a control panel, a medical imaging device may be controlled, for example to start an imaging procedure, pause the imaging procedure, adjust imaging parameters, etc. Another example of such control of a medical imaging device is aborting the imaging procedure, for example in an emergency situation, such as when the medical condition of the patient is deteriorating during the imaging procedure.

Previously, efforts have been made to improve the ease and efficiency with which such a control panel may be operated by a user, for example by making the control panel more ergonomic to use.

However, a drawback of control panels is that they are often provided at a fixed location, e.g., on a workstation connected to the medical imaging device, and even if the control panel is adjustable in terms of location and/or orientation, the adjustability is typically limited. This may be disadvantageous, for example, as in many procedures, the operator may wish to be free in their movement, e.g., to check up on the patient, but may be constrained by the aforementioned fixed location of the control panel.

In US2022/0113809A1, a command communication facility is described, including a sensor unit for detecting movements by an operator, a fixing unit for securing the sensor unit to the operator, an evaluation unit for evaluating the detected movement of the operator and for identifying a command based upon the detected movement, and a command communication unit for transmitting a control command to a medical facility that is to be operated based upon the identified command.

Accordingly, in US2022/0113809A1, the operator may not be tied to a control panel which is arranged at a fixed location.

There has been an advent of medical imaging procedures which do not rely on the presence of an operator. In such procedures, it may be desirable to give the patient at least some degree of control over the medical imaging procedure, e.g., to be able to abort the procedure in case of an emergency situation. Disadvantageously, the approach of US2022/0113809A1 is not well applicable to this setting as the sensor unit, when secured to the patient, may interfere with the medical imaging procedure. Moreover, even when it is the operator who uses the command communication facility of US2022/0113809A1, it is inconvenient for the operator that the sensor unit needs to be secured to the operator.

### SUMMARY OF THE INVENTION

It would be desirable to obtain a system and method which addresses one or more of the aforementioned disadvantages of controlling a medical imaging device.

In a first aspect of the invention, a system is provided for gesture control of a medical imaging device, the system comprising the medical imaging device, the medical imaging device comprising a vibration sensor, the vibration sensor being positioned to detect a gesture performed by a user on a surface of the medical imaging device by detecting a vibration propagated through the surface from the gesture to the vibration sensor, the system further comprising a processor system configured to receive a sensor signal from the vibration sensor and to derive a command signal from the sensor signal to control the medical imaging device.

In a further aspect of the invention, a method is provided for controlling a medical imaging device using gestures, the method comprising performing a gesture on a surface of the medical imaging device by a user, detecting the gesture by the user by a vibration sensor comprised in the medical imaging device by detecting a vibration propagated through the surface from the gesture to the vibration sensor, receiving a sensor signal from the vibration sensor by a processor system, and deriving a command signal from the sensor signal by a processor system.

In a further aspect of the invention, a transitory or non-transitory computer-readable medium is provided comprising data representing instructions, wherein the data, when executed by a processor system, causes a processor system to perform one or more steps of the method, for example any of the computer-implementable steps.

The above measures involve sensing vibrations which are propagated through a surface of a medical imaging device. More specifically, vibrations are sensed which are caused by a user performing a gesture on the surface of the medical imaging device. The gesture may be a movement of a body part in a characteristic way to convey an intent. Performing the gesture on the surface may involve the user contacting the surface of the medical imaging device with the body part, for example with a finger, and moving the body part in the aforementioned characteristic way in relation to the surface so as to convey the intent. Different gestures may be conveyed by different movements, e.g., being different in terms of their characteristics. A characteristic of the movement may for example include a type of movement (e.g., tapping, scratching, sliding, swiping, etc.), a spatial characteristic of the movement (e.g., direction), a temporal characteristic of the movement (e.g., speed, a temporal pattern represented by the movement), and/or an intensity of the movement (e.g., the force or pressure applied to the surface).

To be able to detect the vibrations, the vibration sensor, which for example may be a piezo, a strain, and/or a mechanical sensor, may be positioned on or in a neighborhood of the surface so as to be able to detect the vibrations propagated through the surface. The size of the neighborhood may depend on factors such as the material of the surface, the sensitivity of the vibration sensor, the intensity of gestures, etc. A suitable position may be determined during manufacture of the system.

A processor system is provided which may be configured, e.g., by hardware and/or software, to receive the sensor signal and to derive a command signal from the sensor signal. This may typically involve the processor system analyzing the sensor signal to determine characteristics of the vibration and attributing the characteristics to a particular type of command signal. Typically, distinct types of gestures, e.g., having different movement characteristics, produce distinct types of vibrations, in that the vibrations caused by each of the gestures have different vibrational characteristics. These vibrational characteristics may then be detected by the processor system and attributed to a corresponding command signal. Having determined a particular command signal for a sensor signal, the command signal may then be used to control the medical imaging device. For example, the command signal may be transmitted by the processor system to a control unit of the medical imaging device, or in cases where the processor system also implements the control unit, the command signal may be used internally by the processor system to control the medical imaging device.

In an embodiment, distinct vibration characteristics in the sensor signal are classified using a classifier comprising a deep learning neural network, such as a convolutional neural network.

There are several advantages to the presented system and method. First, a patient may not need to be provided with access to a control panel or sensor unit, which may otherwise interfere with the medical imaging procedure. Rather, an existing surface of the medical imaging device may be employed as an input means. Since such a surface may be within reach of the patient during the imaging procedure, the patient may be enabled to control, to a limited degree, the operation of the medical imaging device. For example, in some embodiments, a limited set of device operations (like 'start', 'pause' or 'abort') may be commanded by the patient themself, which may be beneficial to medical imaging procedures which do not rely on the presence of an operator. Furthermore, since the vibrations propagate from the surface to the vibration sensor, the exact position on where on the surface the gestures are performed is of lesser importance. In other words, a user, such as an operator or the patient, may not need to provide the gesture at a precisely defined location, which provides flexibility during the imaging procedure. For example, the operator may not need to walk back to a control panel to control the medical imaging device but may simply reach to a surface of the medical imaging device. In particular, when attending the patient, such a surface may be nearby as the patient is typically positioned within vicinity of the medical imaging device during the imaging procedure. Furthermore, it is not needed to secure a sensor unit to the operator, which would otherwise require the operator to remember to secure the sensor unit and which may interfere with the imaging procedure if the sensor unit is secured to the patient as operator. Wearable sensors are especially likely to emit radio-frequency signals which may interfere with the imaging procedure, which in turn may negatively affect the quality of the resulting images. Also, metallic parts which are present in many types of sensors may be problematic. In contrast, the vibration sensors are not worn and the locations of the vibration sensors are known, which may avoid or greatly reduce any interference.

In an embodiment, a system is provided wherein the medical imaging device is arranged to receive a patient, the sensor being positioned to detect a gesture by the patient, the surface being arranged in reach of the patient. The medical imaging device may receive the patient, for example using a receiving section such as a bed or a table on which the patient may lie or a bore in which the patient is at least partially inserted. As such, the patient may be in a vicinity of at least part of the medical imaging device, and thereby within vicinity of one or more surfaces of the medical imaging device. This may enable the patient to perform gestures on the surface(s) and thereby control the operation of the medical imaging device, at least to a limited degree. When manufacturing the system, the vibration sensor may be positioned within, or on, or in relation to, the medical imaging device so that vibrations can be received from gestures performed on the surface(s) within reach of the patient when the patient is received by the device.

In an embodiment, a system is provided wherein the command signal starts, stops, and/or changes an intensity of an operation of the medical imaging device. The command signals may thus be associated with certain specific commands to control the device. As such, using different gestures, different aspects of the operation of the medical imaging device may be controlled, in that one gesture may cause an operation to be started and another gesture may cause an operation to be stopped. For example, the entire imaging procedure may be started and stopped through different gestures. This may be especially beneficial in medical imaging procedures where no operator is present, as this may allow the patient themselves to determine when to start the imaging procedure and may allow the patient to stop the imaging procedure in an emergency situation. Specifically, in procedures in which the patient has to assume a specific pose or perform a specific action (e.g., hold their breath fully inhaled), being able to start the procedure with just a gesture may provide the patient with increased autonomy and convenience, which may increase the user-friendliness, effectiveness, and time efficiency of the procedure as a whole. Yet another gesture may cause the intensity of an operation to be changed. This may involve changing one or more imaging parameters. For example, an imaging sequence for an MRI scanner or a dosage of a CT scanner may be adjusted.

In an embodiment, a system is provided wherein the operation of the medical imaging device comprises one or more of: a medical scanning operation, a ventilation operation, an audio playing operation, an emergency stop, and a pause operation. Different gestures may thus trigger a variety of possible actions and procedures at the medical imaging device. While some operations may relate to the imaging procedure itself, other operations which may be controlled by gestures may relate to auxiliary aspects, such as the comfort level of the patient. It may be desirable to ensure sufficient comfort as the comfort level may affect the outcome of the imaging procedure, in that a patient may need to be comfortable enough to be able hold their breath and/or lie still. The comfort may for example be increased by adjusting the ventilation inside the device, by playing-out music, by playing-out anti-noise to cancel out noise generated by the medical imaging device, etc. Another example of an audio playing operation is playing-out instructions for the patient. Moreover, by being able to pause the imaging procedure, the patient may be allowed to change position, breathe for a moment, or sneeze, etc. Also, an operator of the medical imaging device may be a user performing gestures, for whom convenience may be increased as well. An example may be, that a patient-receiving component of the medical imaging device, for example the patient table of an MRI imaging device, may be controlled in its movements, such as a movement to an isocenter of the medical imaging device, based on possible gestures which may be performed by the operator of the medical imaging device. This way, the operator may not have to walk up to the patient table back and forth, to possibly return to a possible control panel or an interface.

In an embodiment, a system is provided wherein the processor system is configured to detect multiple distinct gestures corresponding to multiple respective operations of the medical imaging device. For example, the multiple distinct gestures may exhibit distinct vibration characteristics, corresponding to multiple distinct sensor signals, and the processor system may be configured to detect the multiple distinct sensor signals and derive distinct command signals therefrom, corresponding to multiple respective operations of the medical imaging device. The multiple distinct gestures may comprise distinct physical interactions of the user with the surface of the medical imaging device. Such distinct physical interactions may for example include different types of movement in relation to the surface, for example tapping on the surface, scratching the surface, sliding, stroking, swiping over the surface, etc. Another example is that different body parts may be used to provide different gestures, e.g., fingertips, nails, feet, etc. Yet another example is that the intensity, tempo, pattern, etc., of the physical interaction may be varied to produce different gestures. In some cases, several of the aforementioned variations may be combined to produce different gestures. In general, distinct gestures may be distinct in the vibrations that they produce, for example in the frequency domain and/or in the time domain. These different vibrational characteristics may be detected by the processor system, e.g., using similar techniques as in audio classification, and attributed to respective command signals for the medical imaging device. For example, different vibrational characteristics and the corresponding signals may be distinguished from each other using filtering techniques such as frequency analysis, e.g., using Fourier analysis, or time series analysis, source localization techniques or adaptive noise cancellation. Deep-learning based techniques may be used for the classification of the vibrations in the sensor signal, such as recurrent neural networks, e.g., using long short-term memory units, or an image classifier comprising a convolutional neural network applied to the spectrograms of the vibrations.

In an embodiment, a system is provided wherein the medical imaging device is configured for a breath hold scan and the operation of the medical imaging device comprises a start of the medical scanning operation. A breath hold scan may be an inconvenient procedure for the patient as the patient may be unable to hold their breath for a prolonged amount of time and thus the procedure needs to be timed well to avoid the patient running out of air or starting to breathe too early. By enabling the patient to start the breath hold scan themselves, the patient may decide to start the procedure once they are ready and comfortable. In particular, as the patient can give the gesture only just before starting to hold the breath, the patient may give the start command `just in time' which may result in the time of the patient having to hold their breath being minimized.

In an embodiment, a system is provided wherein the processor system is configured to monitor power consumption of a motor of the medical imaging device, and to derive vibration sensor data therefrom. As such, a sensor monitoring the power consumption of a motor of the medical imaging device may be used as vibration sensor. This may avoid a need for a separate vibration sensor, or such a power consumption sensor may be used as additional sensor. Namely, it has been found that the power consumption of motors may be affected by gestures performed in relation to a surface of the medical imaging device. By monitoring the power consumption, these gestures may be detected or may serve as an additional input source to the detection of gestures.

In an embodiment, a system is provided wherein the processor system is configured to separate from the sensor signal an environment signal, e.g., caused by the operation of the medical imaging device. Vibrations which are present in or caused by the environment may interfere with the sensing of vibrations caused by gestures. However, the influence of such environmental vibrations may be reduced, for example by signal processing, e.g., by separating from the sensor signal the environment signal, by smart placement of the vibration sensor, for example near the expected location where the gestures are provided, by providing multiple sensors and distributing the sensors in relation to the medical imaging devices, or by placing the sensor(s) at positions where fewer environmental vibrations are expected or where there is more isolation from environmental vibrations. Also, filtering techniques employing frequency analysis, Fourier analysis or time series analysis, source localization techniques or adaptive noise cancellation may be used for the purpose of separating the distinct vibrations and the corresponding signals. This may establish a higher signal-to-noise ratio which in turn may increase the sensitivity and/or reliability of detecting vibrations of gestures.

In an embodiment, a system is provided wherein the medical imaging device comprises multiple sensors, and the processor system is configured to localize the gesture using triangulation, for example to localize a source of the gesture and/or to distinguish between different gestures. By being able to localize the gestures, the spatial position and/or orientation of gestures with respect to the surface may be used as an additional distinguishing characteristic of gestures. Additionally, or alternatively, such localization may allow the reliability of the gesture detection to be improved. For example, if a gesture is detected at an unexpected location, or if the location could not be clearly detected, this may indicate that the gesture is detected erroneously, e.g., as a false positive.

In an embodiment, a system is provided wherein the medical imaging device comprises the processor system, or the processor system is comprised in an external signal processing device. The processor system may thus be an internal component of the medical imaging device, for example part of an internal control unit. Alternatively, the processor system may be provided as an external device which is able to communicate with the medical imaging device or with a control unit of the medical imaging device.

In a further aspect of the invention, a medical imaging device is provided comprising the vibration sensor and processor system as described in this specification.

A person skilled in the art will appreciate that the system and method may be applied to medical imaging devices which acquire multi-dimensional image data, e.g., two-dimensional (2D), three-dimensional (3D) or four-dimensional (4D) images, for example using various acquisition modalities such as, but not limited to, standard X-ray Imaging, CT, MRI, Ultrasound (US), PET, SPECT, and Nuclear Medicine (NM).

The embodiments mentioned above are described in the accompanying claims. Further, specific embodiments are set forth in the dependent claims.

### BRIEF DESCRIPTION OF DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,
Fig. 1 schematically shows an embodiment of a system for gesture control of a medical imaging device,
Fig. 2 schematically shows an embodiment of an MRI imaging device with gesture control,
Fig. 3 schematically shows a processor system configured to receive a sensor signal from the vibration sensor and to derive a command signal from the sensor signal to control the medical imaging device,
Fig. 4 schematically shows an example of a flowchart of a method for controlling a medical imaging device using gestures,
Fig. 5a schematically shows a computer readable medium having a writable part comprising a computer program according to an embodiment,
Fig. 5b schematically shows a representation of a processor system according to an embodiment.

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

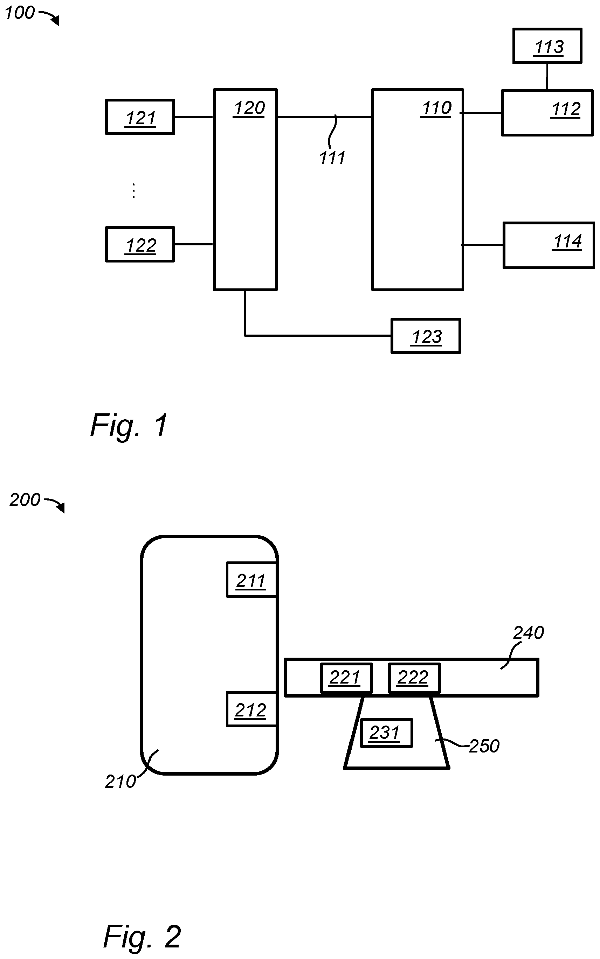

Fig. 1 schematically shows an embodiment of a system 100 for gesture control of a medical imaging device 110. The system 100 may comprise the medical imaging device 110 and a processor system 120. Fig. 1 shows the processor system 120 separately from the medical imaging device 110. For example, the processor system 120 may be comprised in an external signal progressing device, for example an external control unit for the medical imaging device 110. However, this is not a limitation, as the processor system 120 may also be part of the medical imaging device 110, e.g., an internal component of the medical imaging device.

The system 100 may enable the sensing of vibrations which are propagated through one or multiple surfaces of the medical imaging device 110. In particular, vibrations may be sensed which are caused by a user performing a gesture on the one or more surfaces of the medical imaging device 110. To sense the vibrations, the medical imaging device 110 may comprise one or multiple vibration sensors 121,122. The one or more vibration sensors 121,122 may, although shown separately from the medical imaging device 110, be internal components of the medical imaging device 110. Alternatively, the one or more vibration sensors 121,122 may be external components which may for example be affixed to a surface of the medical imaging device 110. The one or more vibration sensors 121,122 may be configured to detect gestures performed by the user on the one or more surfaces of the medical imaging device 110 by sensing vibrations propagated through the one or more surfaces which are caused by a respective gesture. In particular, to be able to detect the vibrations, the one or more vibration sensors 121,122 may be positioned in a neighborhood of the one or more surfaces so as to be able to detect vibrations propagated through the one or more surfaces. The size of the neighborhood, and thus the maximum distance between the one or more surfaces and the one or more vibration sensors may depend on factors such as the material of the one or multiple surfaces, the sensitivity of the one or more vibration sensors, the intensity of gestures, etc.

A suitable position for the one or more vibration sensors 121,122 may be determined during manufacture of the system 100, for example by performing gestures on the one or more surfaces and determining at which positions the one or more vibration sensors 121,122 are still able to sense the vibrations, e.g., with a sufficient signal-to-noise ratio.

Non-limiting examples of suitable vibration sensors 121,122 include piezo-based vibration sensors, strain sensors, such as strain gauges, mechanical vibration sensors, etc. It is noted that, although not explicitly shown in Fig. 1, the one or more vibration sensors 121,122 may typically be integrated in or placed on a surface of the medical imaging device 110, e.g., in direct or indirect contact with the one or more surfaces on which the user is expected to provide the gesture(s). However, this is not a limitation, as in some examples, vibration sensors 121,122 arranged outside of the medical imaging device 110 may still be able to sense vibrations propagated through the one or more surfaces of the imaging device 110.

The processor system 120 may be configured, e.g., by hardware and/or software, to receive a sensor signal from the one or more vibration sensors 121,122, and to derive a command signal 111 from the sensor signal. This may typically involve the processor system 120 analyzing the sensor signal to determine characteristics of the vibration and attributing the characteristics to a particular type of command signal 111. It is noted that if several sensor signals are received, e.g., from different vibration sensors 121,122, the sensor signals may be jointly analyzed to determine the characteristics of the vibration and jointly attributed to a particular type of command signal 111.

Typically, distinct types of gestures, e.g., having different movement characteristics, produce distinct types of vibrations, in that the vibrations caused by each of the gestures may have different vibrational characteristics. These different vibrational characteristics may then be detected by the processor system 120 and attributed to different command signals 111 depending on the characteristics of the vibrations in the sensor signal. For example, the processor system 120 may use heuristics or machine-learning based techniques to detect different gestures by classifying the vibrations comprised in the sensor signal based on their characteristics. For example, similar techniques may be used as in the field of audio classification, where frequency-domain and/or time-domain based techniques may be used to distinguish between different audio classes, e.g., between the sound of a dog and the sound of a cat. In a specific example, deep-learning based techniques may be used for the classification of the vibrations in the sensor signal, such as for example recurrent neural networks, e.g., using long short-term memory units, or convolutional-neural-network-based image classifiers applied to a spectrograms of the vibrations.

Having determined a particular command signal 111 for a sensor signal, the command signal 111 may then be used to control the medical imaging device 110. For example, the command signal 111 may be transmitted by the processor system 120 to a control unit of the medical imaging device 110, or in cases where the processor system 120 also implements the control unit, the command signal 111 may be used internally by the processor system 120 to control the medical imaging device 110. For example, different command signals 111 may start, stop, and/or change an intensity of an operation of the medical imaging device 110. Examples of such operations include, but are not limited to, a medical scanning operation, an emergency stop operation, and a pause operation. Also, the movements of a patient-receiving component of the medical imaging device 110, for example the patient table of an MRI imaging device, may be gesture-controlled. These movements may be controlled by an operator of the medical imaging device 110, who may be a user of the system 100, and thus may perform gestures, such as swiping over an operator interface: a possible command may then be, that the patient-receiving component, may move to an isocenter of the medical imaging device.

With continued reference to the gesture performed on the surface of the medical imaging device 110, it is noted that such a gesture may be a movement of a body part in a characteristic way to convey an intent. Performing the gesture on the one or more surfaces may involve the user contacting the one or more surfaces of the medical imaging device 110 with a body part, for example a finger, and moving the body part in the aforementioned characteristic way in relation to the one or more surfaces so as to convey the intent. Different gestures may be conveyed by different movements, e.g., being different in terms of their characteristics. A characteristic of the movement may for example include a type of movement (e.g., tapping, scratching, sliding, swiping, etc.), a spatial characteristics of the movement (e.g., direction), a temporal characteristics of the movement (e.g., speed, a temporal pattern represented by the movement), and/or an intensity of the movement (e.g., the force or pressure applied to the one or multiple surfaces). Examples of different types of gestures may include tapping on a surface, scratching the surface, sliding on the surface, stroking the surface, or swiping the surface. In a specific example, tapping may be performed using fingertips, scratching using fingernails, and swiping, stroking, and sliding using any part of fingers. Instead of or in addition to employing hands for performing the gestures, other parts of the body may be employed, such as a foot, knee, upper arm, or elbow. The processor system 120 may be configured to detect the vibration characteristics of such different types of gestures to be able to attribute a vibration in the sensor signal to a particular type of command signal 111.

In some embodiments, the medical imaging device 110 may comprise at least three vibration sensors 121,122 and the processor system 120 may be configured to localize the gestures using triangulation. Such triangulation is known per se. For example, triangulation may be used to detect a location of an earthquake from vibrations (seismic waves). By being able to localize the gestures, the spatial position and/or orientation of gestures with respect to the one or multiple surfaces may be used as an additional distinguishing characteristic of gestures. Additionally, or alternatively, such localization may allow the reliability of the gesture detection to be improved. For example, if a gesture is detected at an unexpected location, or if the location could not be clearly detected, this may indicate that the gesture is detected erroneously, e.g., as a false positive.

A motor sensor 113 may monitor the power consumption of a motor 112 of the medical imaging device 110. In some embodiments, such a motor sensor 113 may be used as vibration sensor. For example, the motor 112 may actuate a moving part of the medical imaging device 110, such as a patient table. Gestures may cause vibrations to propagate through the moving part. These vibrations may then be registered by the motor sensor 113, for example as deviations from a nominal current. By using such a motor sensor 113, a separate vibration sensor 121,122 may not be needed, or such a motor sensor 113 may be used as an additional sensor in conjunction with other vibration sensor(s).

In some embodiments, an auxiliary system 114 may be provided to regulate conditions or properties of or inside the medical imaging device 110, such as ventilation or audio, and through different gestures, an operation of the auxiliary system 114 may be controlled. For example, the processor system 120 may be configured to control the ventilation and/or the audio-playout by the auxiliary system 114. In a specific example, through gestures, the ventilation inside the medical imaging device 110 may be adjusted, for exampling by controlling a fan blowing inside the medical imaging device, or music may be played-out, or anti-noise may be played-out to cancel out noise generated by the medical imaging device 110, or instructions for the patient may be played-out, etc.

In some embodiments, the system 100 may comprise an environment sensor 123. This may relate to the following. The processor system 120 may be configured to separate an environment signal from the sensor signal. The environment signal may represent vibrations which are present in or caused by the environment and which may interfere with the sensing of vibrations caused by gestures. The influence of such environmental vibrations may be reduced, for example by signal processing, e.g., by separating the environment signal from the sensor signal. This signal separation may for example be performed using filtering techniques employing frequency analysis, such as Fourier analysis, or time series analysis, source localization techniques or adaptive noise cancellation. Additionally, or alternatively to such signal processing, the presence of vibrations in the environment may be detected by the aforementioned environment sensor 123. This sensor 123 may be specifically configured to be insensitive to the vibrations caused by gestures, for example by being provided at a position where vibrations of gestures cannot easily reach the environment sensor. In a specific example, the environment sensor 123 may be a microphone to record the machine vibrations which are not caused by the user performing the gestures. In another example, one of the multiple vibration sensors 121,122 may function as an environment sensor 123, e.g., by the vibration sensor being positioned near where the influence of environmental vibrations is large or largest.

It is noted that the use of an environment sensor 123 may also be avoided, e.g., by appropriate placement of the vibration sensors 121,122, for example near the expected location where the gestures are provided, and/or by placing the sensor(s) 121,122 at positions where fewer environmental vibrations are expected. In yet another example, the environment signal may be separated from the sensor signal using the aforementioned signal processing techniques. For example, the sensor signal may not only pick-up vibrations from gestures but also motor noise. The signals corresponding to the gestures may be separated from the motor noise using, for example, filtering techniques employing Fourier or frequency analysis (e.g., using a band-pass filter) or time series analysis (e.g., by means of a deletion of the average vibration characteristics of the motor). As the vibration characteristics of the motor noise may be known, the motor noise can be filtered out or at least reduced so as to improve the signal-to-noise ratio of the vibration signal. Another option may be adaptive noise cancellation, in which the aforementioned environment sensor may be arranged and configured to receive the vibrations stemming from the motor, but not the vibrations corresponding to the performed gestures.

Fig. 2 shows an MRI imaging device 200. The MRI imaging device 200 may comprise a scanning component 210, a patient table 240, a patient table base 250, and vibration sensors 211-231. The MRI imaging device 200 may be controllable by gestures which, as in the example of Fig. 1, may be detected by vibration sensors and identified in the sensor signals by a processor system (the processor system is not shown in Fig. 2). The scanning component 210 may comprise a bore to receive a patient. The patient table 240, also called a patient bed, may comprise a movable platform that slides into and out of the bore 210. The patient may lie down on the table 240 and may be moved into the bore 210 for a scan. Typically, the table 240 may be adjusted for height and may be moved horizontally to correctly position the patient inside the MRI imaging device 200.

One or more vibration sensors 211-231 may be placed at various positions on and inside the MRI imaging device 200. For example, one or more vibration sensors 211,212 may be placed inside the bore 210 to sense vibrations caused by gestures performed on the inside of the bore of the scanning component 210. Additionally, or alternatively, vibration sensors may be provided in or on the patient table 240 or in or on the base 250 of the patient table to be able to pick-up vibrations caused by gestures performed on a surface of the patient table 240 and/or the base 250. It is noted that the locations of the vibration sensors 211-231 shown in Fig. 2 are merely exemplary and may depend on which one or more surfaces are to be used as input means for gestures by the user. For example, the location of the vibration sensors 211-231 may depend on whether the patient, an operator or both are expected to provide gestures as input.

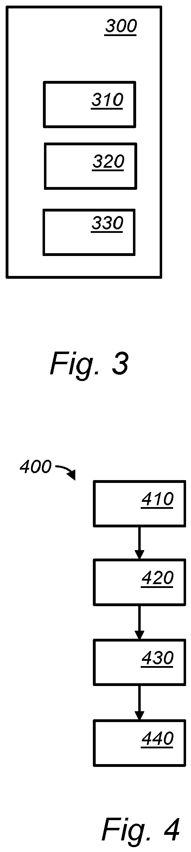

Fig. 3 schematically shows a processor system 300 configured to receive a sensor signal from the vibration sensor and to derive a command signal from the sensor signal to control the medical imaging device. The processor system 300 may comprise a processor 310, a storage 320 and a communication interface 330. Briefly speaking, the processor 310 may be used by the processor system 300 to perform any processing tasks described in this specification in relation to the processor system, the storage 320 may be used for temporary storage, and the communication interface 330 may be used to communicate with external entities, such as sensors, other processor systems, etc.

The processor 310 may for example be a known type of processor circuit, such as a microprocessor, CPU, GPU, etc. The processor system 300 may also comprise multiple processors. A processor circuit may be implemented in a distributed fashion, e.g., as multiple sub-processor circuits. For example, the processor system 300 may use cloud computing. The processor may be configured to execute appropriate software stored at the processor system 300; for example, the software may have been downloaded and/or stored in a corresponding memory, e.g., a volatile memory such as RAM or a non-volatile memory such as Flash. Instead of using software to implement a function, the processor 310 may, in whole or in part, be implemented in programmable logic, e.g., as field-programmable gate array (FPGA). The processor 310 may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In hybrid embodiments, functional units are implemented partially in hardware and partially in software stored and executed on the processor system 300.

Embodiments of the communication interface 330 may include one or more of the following. The communication interface may comprise a network interface to a local or wide area network, e.g., the Internet, a storage interface to an internal or external data storage, such as an internal hospital storage where hospital- or patient-specific data may be saved, stored, and shared, or an application interface (API), etc. The communication interface 330 may also comprise a sensor interface and/or a control interface. The processor system 300 may thus receive sensor signals and transmit command signals to the medical imaging device via the communication interface 330.

The storage 320 may be, e.g., electronic storage, magnetic storage, etc. The storage may comprise local storage, e.g., a local hard drive or electronic memory. The storage 320 may comprise non-local storage, e.g., cloud storage. In the latter case, the storage 320 may comprise a storage interface to the non-local storage. The storage may comprise multiple discrete sub-storages together making up the storage 320 .

The storage 320 may be non-transitory storage. For example, the storage 320 may store data in the presence of power such as a volatile memory device, e.g., a Random Access Memory (RAM). For example, the storage 320 may store data in the presence of power as well as outside the presence of power such as a non-volatile memory device, e.g., Flash memory. The storage 320 may comprise a volatile writable part, say a RAM, a non-volatile writable part, e.g., Flash. The storage may comprise a non-volatile non-writable part, e.g., ROM, e.g., storing part of the software.

In some examples, where the processor system 300 is part of the medical imaging device, the aforementioned processor 310, storage 320, and communication interface 330 may be components of the medical imaging device. With continued reference to the medical imaging device, it is noted that, although not shown in Fig. 3, the medical imaging device may additionally have a user interface, which may include well-known elements such as one or more buttons, a keyboard, display, touch screen, etc.

Fig. 4 schematically shows an example of an embodiment of a method for controlling a medical imaging device using gestures. A step 410 may comprise performing a gesture on a surface of the medical imaging device by a user. A step 420 may comprise detecting the gesture by the user by a vibration sensor comprised in the medical imaging device by detecting a vibration propagated through the surface from the gesture to the vibration sensor. A step 430 may comprise receiving a sensor signal from the vibration sensor by a processor system. A step 440 may comprise deriving a command signal from the sensor signal by the processor system. Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started. Embodiments of the method may be executed using software, which comprises instructions for causing a processor system to perform steps 420, 430, 430 of method 400. Software may only include those steps taken by a particular sub-entity of the system. The software may be stored in a suitable storage medium, such as a hard disk, a memory, an optical disc, etc. The software may be sent as a signal along a wire, or wireless, or using a data network, e.g., the Internet. The software may be made available for download and/or for remote usage on a server.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

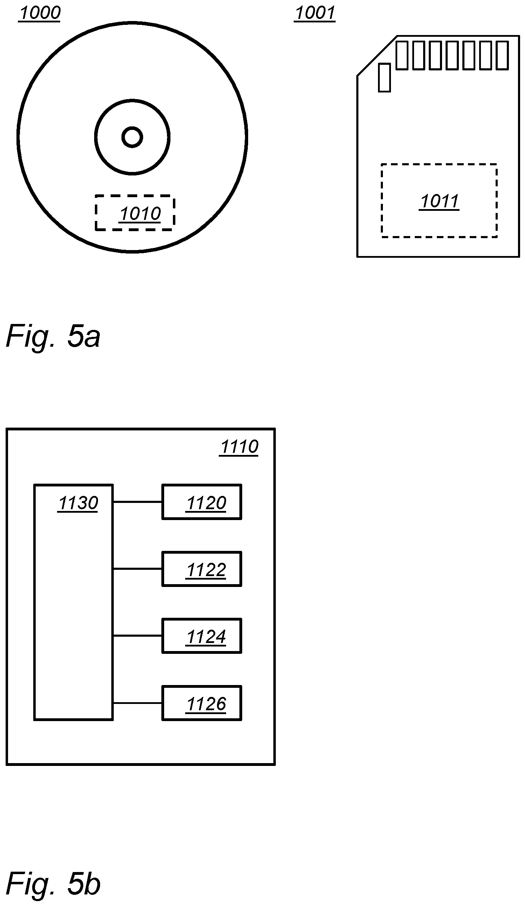

Fig. 5a shows a computer readable medium 1000 having a data 1010 stored thereon, and a computer readable medium 1001 also having a data 1011 stored thereon. Computer readable medium 1000 is shown in the form of an optically readable medium. Computer readable medium 1001 is shown in the form of an electronic memory, in this case a memory card. The stored data 1010 and 1011 may represent computer programs which may be comprised of instructions, which when executed by a processor system, cause a processor system to perform an embodiment of a method, according to an embodiment as described in Fig. 4. The computer programs 1010 and 1011 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer programs 1010 and 1011 comprise instructions for causing a processor system to perform an embodiment of said method of controlling a medical imaging device using gestures.

Fig. 5b shows a schematic representation of a processor system 1110 according to an embodiment. The processor system 1110 comprises a processing unit 1120, e.g., a CPU, for running computer program components to execute a method according to an embodiment and/or implement its modules or units. The processor system 1110 comprises a memory 1122 for storing programming code, data, etc. Part of memory 1122 may be read-only. The processor system 1110 may comprise a communication element 1126, e.g., an antenna, connectors or both, and the like. The processor system 1110 may comprise a dedicated integrated circuit 1124 for performing part or all of the processing defined in the method. The processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

While system 1110 is shown as including one of each described component, the various components may be duplicated in various embodiments. For example, the processing unit 1120 may include multiple microprocessors that are configured to independently execute the methods described herein or are configured to perform steps or subroutines of the methods described herein such that the multiple processors cooperate to achieve the functionality described herein. Further, where the system 1110 is implemented in a cloud computing system, the various hardware components may belong to separate physical systems. For example, the processing unit 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claims.

### Reference signs list

The following list of references and abbreviations is provided for facilitating the interpretation of the drawings and shall not be construed as limiting the claims.
- 100: system for gesture control of a medical imaging device
- 110: medical imaging device
- 111: command signal
- 112: motor
- 113: motor sensor
- 114: auxiliary system
- 120: processor system
- 121-122: vibration sensor
- 123: environment sensor

- 200: MRI imaging device
- 210: scanning component
- 211-231: vibration sensors
- 240: patient table
- 250: patient table base

- 300: processor system
- 310: processor
- 320: storage
- 330: communication interface

- 400: method for controlling a medical imaging device using gestures
- 410: performing a gesture on a surface of the medical imaging device
- 420: detecting the gesture using a vibration sensor
- 430: receiving a sensor signal from the vibration sensor
- 440: deriving a command signal from the sensor signal

- 1000, 1001: computer-readable medium
- 1010, 1011: stored data
- 1110: processor system
- 1120: processing unit
- 1122: memory
- 1124: dedicated integrated circuit
- 1126: communication element
- 1130: interconnect

## Claims

1. A system (100) for gesture control of a medical imaging device (110), the system comprising
- the medical imaging device (110,200), the medical imaging device (110,200) comprising a vibration sensor (121,122,211-231), the vibration sensor (121,122,211-231) being positioned to detect a gesture performed by a user on a surface of the medical imaging device (110,200) by detecting a vibration propagated through the surface from the gesture to the vibration sensor (121,122,211-231); and
- a processor system (120,300) configured to receive a sensor signal from the vibration sensor (121,122,211-231), and to derive a command signal (111) from the sensor signal to control the medical imaging device (110,200).

2. A system (100) as in claim 1, wherein the medical imaging device (110,200) is arranged to receive a patient, the vibration sensor (121,122,211-231) being positioned to detect a gesture by the patient, the surface being arranged in reach of the patient.

3. A system (100) as in any of the previous claims, wherein the command signal (111) starts, stops, and/or changes an intensity of an operation of the medical imaging device (110,200).

4. A system (100) as in claim 3, wherein the operation of the medical imaging device (110,200) comprises one or more of: a medical scanning operation, a ventilation operation, an audio playing operation, an emergency stop, and a pause operation.

5. A system (100) as in claim 3, wherein the medical imaging device (110,200) is configured for a breath hold scan and the operation of the medical imaging device (110,200) comprises a start of the medical scanning operation.

6. A system (100) as in any of the preceding claims, wherein the processor system (120,300) is configured to detect multiple distinct gestures corresponding to multiple respective operations of the medical imaging device (110,200).

7. A system (100) as in claim 6, wherein the processor system (120,300) is configured to detect the multiple distinct gestures by detecting distinct vibration characteristics in the sensor signal and to attribute each of the distinct vibration characteristics to a distinct command signal (111), corresponding to multiple respective operations of the medical imaging device (110,200).

8. A system (100) as in any of the previous claims, wherein the vibration sensor (121,122,211-231) is connected to the processor system (120,300) through a wire.

9. A system (100) as in any of the previous claims, wherein the vibration sensor (121,122,211-231) comprises a piezo, a strain, and/or a mechanical sensor.

10. A system (100) as in any of claims 1-8, wherein the processor system (120,300) is configured to monitor power consumption of a motor (112) of the medical imaging device (110), and to derive vibration sensor data therefrom.

11. A system (100) as in any of the previous claims, wherein the gestures by the user comprise one or more of the following: tapping commands, stroking commands, sliding commands, and swiping commands.

12. A system (100) as in any of the previous claims, wherein the vibration sensor (121,122,211-231) is positioned to detect a gesture by the hand and/or foot of a user.

13. A system (100) as in any of the previous claims, wherein the medical imaging device (110,200) is one of an MRI scanner, a CT scanner, a SPECT scanner, and a PET scanner.

14. A system (100) as in any of the previous claims, wherein the processor system (120,300) is configured to separate from the sensor signal an environment signal, e.g., caused by the operation of the medical imaging device (110,200).

15. A system (100) as in any of the previous claims, wherein the medical imaging device (110,200) comprises multiple sensors (121,122,211-231), and the processor system (120,300) is configured to localize the gesture using triangulation.

16. A system (100) as in any of the previous claims, wherein
- the medical imaging device (110,200) comprises the processor system (120,300); or
- the processor system (120,300) is comprised in an external signal processing device.

17. A method (400) for controlling a medical imaging device (110,200) using gestures, the method (400) comprising
- performing (410) a gesture on a surface of the medical imaging device (110,200) by a user;
- detecting (420) the gesture by the user by a vibration sensor (121,122,211-231) comprised in the medical imaging device (110,200) by detecting a vibration propagated through the surface from the gesture to the vibration sensor (121,122,211-231);
- receiving (430) a sensor signal from the vibration sensor (121,122,211-231) by a processor system (120,300); and
- deriving (440) a command signal (111) from the sensor signal by the processor system (120,300).

18. A transitory or non-transitory computer-readable medium (1000) comprising data (1020) representing instructions, which when executed by a processor system, cause the processor system to perform one or more steps of the method according to claim 17.
